# EUROPEAN PATENT APPLICATION

(11) **EP 1 199 055 A1**
(43) Date of publication of application: **24.04.2002**
(21) Application number: 01124723.6
(22) Date of filing: 16.10.2001
(51) Int. Cl.: A61F 9/013, B23P 15/40, A61B 17/32, B26B 9/00, B26B 21/00, B26B 21/58, B21D 53/64

(54) **Blade with amorphous cutting edge**

(30) Priority: 16.10.2000 DE 10051215; 14.06.2001 US 881320
(71) Applicant: Gebauer GmbH, 75242 Neuhausen (DE)
(72) Inventor: Gebauer, Detlev P., Dipl.-Ing. (FH), 75233 Tiefenbrunn (DE)
(74) Representative: Leson, Thomas Johannes Alois, Dipl.-Ing.

(57) **Abstract**

The invention relates to a blade (1) consisting of a carrier member (7) and a foil (5) of amorphous metal joined to the carrier member via an adhesive layer (9), said amorphous metal forming the cutting edge (3) of the blade. The cutting edge (3) of the blade is formed by a one-sided ground section extending over the carrier material as well as the amorphous metal. The use of the foil (5) of amorphous metal allows the manufacture of an inexpensive blade having a high sharpness and a, with respect to the blade body, exactly defined position of the cutting edge (3).

## Description

### Field of the Invention

The invention relates to a blade, in particular to a surgical blade, having an amorphous cutting edge as well as to a method for manufacturing the same.

### Background of the Invention

Up to now, a variety of different materials from stainless steel to diamond have been proposed for the cutting edge of blades.

Among the known materials, diamond has the best cutting capacity due to its great hardness because the cutting edge can be ground with a very small radius of curvature lying in the nanometer range. Disadvantages are, however, the high material cost and the difficulties in applying the diamond as cutting edge on a knife.

A blade made of stainless steel, on the other hand, can be manufactured in a comparatively simple way, and offers considerable cost advantages. However, steel is comparatively soft and, due to its crystalline structure, lets itself be sharpened to a restricted extent only. Due to the inhomogeneities in the form of crystal grains, no radius of curvature of less than several ten nanometers can be realized on the cutting edge as a rule.

Surprisingly good results have been achieved with blades the cutting edges of which consist of an amorphous metal.

The EP-A-0 119 714 and the WO 86/02868 propose to melt the cutting edge of a metallic blade body by laser beam treatment and to rapidly cool it off in a water bath. In this way, the cutting edge is amorphized.

Not only the cited EP-A-0 119 714, but also the German laid-open document 2 362 895 indicates as further possibility to provide the cutting edge area of a cutting tool with an amorphous metal film by means of a coating process.

Furthermore, the EP-A-0 467 937 discloses a surgical blade consisting of an approximately 100 µm thick strip of amorphous metal, said strip having been sharpened on both sides and being coated with electrochemically deposited nickel layers of approximately 5 to 30 µm thickness on the cover faces parallel to the cutting edge.

### Summary of the Invention

It is among the objectives of the invention to provide a blade having improved properties and being particularly suited for surgical purposes, and a manufacturing method for said blade.

These objectives are, for example, accomplished by a blade comprising a carrier portion and a thin-walled cover portion made of amorphous metal, which is joined to the carrier portion, the amorphous metal of the cover portion forming a cutting edge of said blade.

The carrier portion and the cover portion of the blade can be formed by a foil made of amorphous metal being joined to a carrier member in order to form a blade body, and by the blade body being ground such that the amorphous metal forms a cutting edge.

A foil made of amorphous metal is to be understood as a material commercially offered in the form of thin amorphous metal tapes as semi-finished products. The manufacturing methods for such amorphous metal tapes are well-known. A preferred method for producing amorphous metal tapes of good quality is a rapid solidification process. In this case a flow of the molten substance is cast onto a rotating metal cylinder being cooled from the inside, so that the molten substance rapidly solidifies at a cooling rate of 10⁵ to 10⁷ K/s or more in the shape of a thin film metal tape.

As amorphous metals, usually compositions of the MₐX_{b} kind are used, M standing for one or more elements from Ni, Fe, Co, Cr, and V and X for one or more elements from P, B, C, Si, Al, Sb, Sn, In, Ge, and Be, and a amounting to 65 to 90 at% and b to 10 to 35 at%. The elements listed under M can also partly be replaced by Mo, Mn, Ti, W, and Co.

The amorphous metal tape used for the foil need not be completely amorphous, but may partly also have crystalline regions. In case of the rapid solidification process, the side of the metal tape facing the cooling cylinder is cooled more rapidly than the opposite side of the metal tape, so that it generally has a higher amount of amorphous phase. The higher the amount of amorphous regions, the higher is, as a rule, the hardness and homogeneity of the amorphous metal tape. Amorphous metal tapes obtained by rapid solidification and having good quality usually have a thickness of between 10 and 50 µm and a width of less than 75 mm.

Using a foil of amorphous metal has a number of advantages over conventional blades. While, for blades having a crystalline metallic cutting edge region, the homogeneity of the cutting edge is adversely affected by the presence of crystal grains, the homogeneity of the cutting edge of the blade according to the invention is considerably higher due to the more even amorphous structure. The high homogeneity of the foil made of amorphous metal, which forms the cutting edge, allows to achieve a radius of curvature at the cutting edge of the blade which is smaller by far than that of conventional steel blades. Thus, the blade according to the invention is sharper.

Despite the excellent quality of the cutting edge, the manufacturing cost of the blade according to the invention is considerably lower than that of a diamond cutting edge. The use of the amorphous metal tape available as semi-finished product allows to save cost for the manufacture of the blade according to the invention as against a blade where the amorphous cutting edge region is achieved by the application of an amorphous layer onto a metallic base material or by amorphization of a metallic base material. Besides, an amorphous metal in the shape of a tape or foil, even if it has some crystalline regions, is usually more homogeneous than an amorphous metal manufactured by amorphization or coating and, therefore, has better properties.

The cutting edge of the above-described blade is preferably formed by an one-sided ground section. It is possible to perform grinding on only one side because the amorphous metal has a high hardness and because it forms a cover portion of the blade. The high hardness of the amorphous metal prevents the formation of a burr during grinding, which serves to avoid a counter-grinding required for removing the burr. Thus, the cutting edge of the blade can be formed by the ground surface and by the cover face of the blade formed by the cover portion, so that the position of the cutting edge with respect to the blade body is exactly defined.

The grinding can be performed such that the ground section extends over the carrier member as well as the foil of amorphous metal. However, in view of the possibility of grinding out particles of the carrier member which may damage the foil of amorphous metal, it is preferable to grind only the foil of amorphous metal. Optionally, it is also possible to form a multi-faced ground section with the ground surface of the foil of amorphous metal having a greater interior grinding angle than an adjacent ground surface or with the ground surface of the foil of amorphous metal having an offset in respect of an adjacent ground surface.

Further, it is preferable that the cover face of the amorphous metal foil which has a higher amount of amorphous phase forms the cutting edge. In this case the homogeneity and hardness of the cutting edge are further improved.

In order to join the foil of amorphous metal to the carrier member, an adhesive process is advantageous. Since the adhesion can be performed without a temperature load, it is possible to avoid a change in the material properties of the amorphous metal.

A particularly cheap manufacturing method for the above-described blade results when the carrier member and the foil of amorphous material are provided in a tape shape, which can be wound up and off, and are joined to form a multi-layer tape from which the blade bodies to be ground can be formed by punching in contours. For the time being, the blade bodies can remain connected at residual links, so that the tape does not get interrupted.

Hardened high-grade steel is recommendable as material for the tape-shaped carrier member, and should have a thickness of preferably 150 to 350 µm for reasons of rigidity. The rigidity of the blade can be increased by curving or folding the blade after grinding alongside the cutting edge.

There is, moreover, the possibility to magnetically encode the above-described blade. For this, the tape of amorphous metal should be soft magnetic and should have a remanence ratio, i.e. a ratio between remanence and saturation magnetization, of more than 0.7. A high remanence ratio means that the material has a narrow, definedly adjusted magnetic hysteresis curve. The magnetic encoding is comparable to that of labels used for an electronic goods protection system where foils of amorphous metal are used, as well.

The blade according to the invention is particularly suited for being used as scalpel, microkeratome, or microtome.

The above-indicated and further technical solutions with their features and advantages will become further apparent upon consideration of the following description of preferred embodiments, taken in conjunction with the accompanying drawings, wherein:
Fig. 1 is a plan view of a blade according to a first embodiment;
Fig. 2 is a partial sectional view along the line A-A in Fig. 1, showing the multi-layer structure of the blade body of the blade;
Fig. 3 is a partial sectional view along the line B-B in Fig. 1, showing the cutting edge area of the blade;
Fig. 4 is an example of using the blade according to the first embodiment in a known device for corneal surgery;
Fig. 5 is a perspective view of a blade according to a second embodiment;
Fig. 6 is a perspective view of a blade according to a third embodiment;
Fig. 7 is an exploded view showing the components of the blade in Fig. 6; and
Fig. 8 is a partial sectional view along the line C-C in Fig. 6, showing the cutting edge area of the blade.

### Description of the Preferred Embodiments

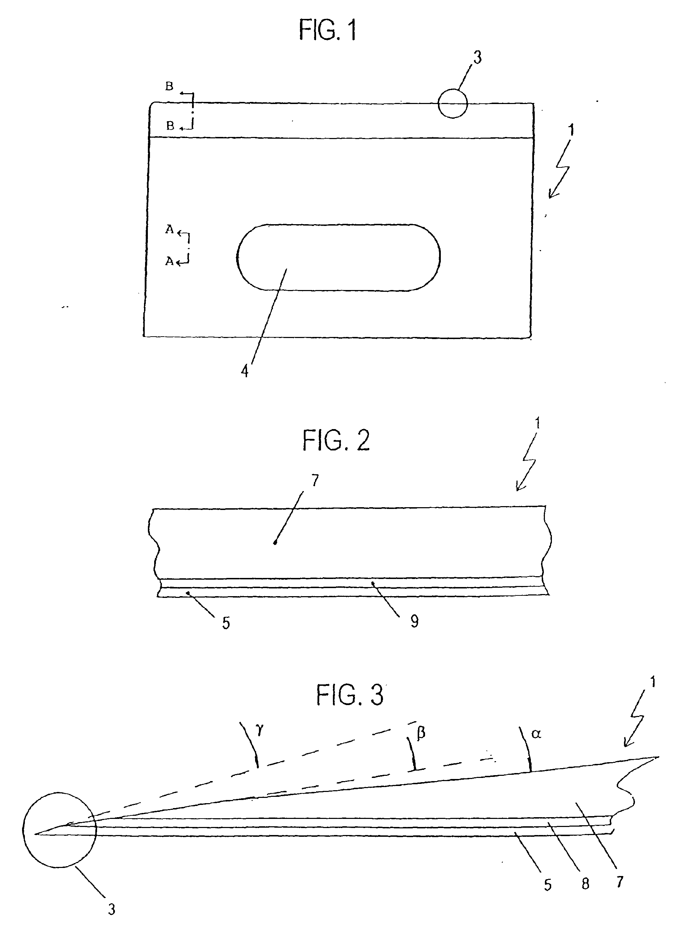

Referring now to Figs. 1 to 3, a first embodiment of a blade is described which is particularly suited as microkeratome, i.e. as knife for operations of the cornea.

As can be seen in Figs. 1 to 3, a blade 1 consists of a carrier member 7 and a foil 5 made of amorphous metal and joined to said carrier member, said amorphous metal forming a cutting edge 3. The blade 1 is formed as an elongated cutting body on the wider side of which the cutting edge 3 is formed. The blade 1 has a width of about 13.4 mm, a length of about 8 mm and a thickness of about 0.25 mm. The blade body comprises an elongated hole 4 spaced apart from the cutting edge 3 and extending transversely thereto. The elongated hole 4 serves to receive a support, which is not shown, by which the blade 1 can be guided. The elongated hole 4 has a dimension of about 4.9 mm in the direction of the width of the blade, and a dimension of about 2.2 mm in the direction of its length.

Fig. 2 shows a sectional view through the blade body of the blade 1 along the line A-A shown in Fig. 1. As can be recognized in Fig. 2, the blade 1 has, in its direction of thickness, a multi-layer construction comprising the foil 5 of amorphous metal and the comparatively thick, strip-shaped carrier member 7, the flat surfaces of which are bonded via an adhesive layer 9. Thus, the carrier member 7 forms a carrier portion, and the foil 5 of amorphous metal a cover portion of the blade 1.

A thin, soft magnetic metal tape has been used for the foil 5 of amorphous metal, said tape being available as semi-finished product from the company Vakuumschmelze of Hanau, Germany under the trademark name "Vitrovac". Besides non-metallic elements, this material substantially consists of cobalt, and is gained from the molten substance by rapid solidification. The amorphous material has a thickness of about 20 to 25 µm and is noted for a high mechanical hardness as well as a narrow, definedly adjusted magnetic hysteresis curve having a remanence ratio of more than 0.7.

The cover face of the metal tape being more rapidly cooled during rapid solidification and having a higher amount of amorphous phase is arranged on the outside of the multi-layer construction of the blade.

The adhesive layer 9 consists of cyanoacrylate, and has a thickness of about 25 to 30 µm. Due to the presence of the adhesive layer 9, the foil 5 of amorphous metal is magnetically non-interacting with the carrier member 7, an about 200 µm thick strip of hardened high-grade steel.

As shown in Fig. 3, which represents a section through the cutting edge area of the blade 1 along the line B-B shown in Fig. 1, the blade body of the blade 1 is ground on one side from the side of the carrier material 7. The one-sided ground section is stepped and extends over the carrier material 7 as well as the adhesive layer 9 and the amorphous metal 5, the amorphous metal of the foil 5 forming the cutting edge 3.

As can be seen in Fig. 3, the cutting edge area of the blade 1 shows a three-faced ground section. The carrier member 7 is provided with a rough-ground section having an internal angle of α=5°. The rough-ground section verges into a ground section having an internal angle of β=9° which also extends over the adhesive layer 9 in addition to the carrier member 7. The cutting edge 3 is finally formed by a honed section having an internal angle of γ=17°, which only extends over the foil 5 of amorphous metal.

Due to the high homogeneity and hardness of the amorphous metal forming the cutting edge 3, the blade 1 constructed in this way has a high sharpness which is close to that of a diamond blade. Thus, the blade according to the invention constitutes an excellent alternative to conventional blades.

The blade is magnetically encoded by the small, definedly set hysteresis curve of the foil 5 of amorphous and soft magnetic metal. The magnetic encoding can be detected by a suitable reading unit, which opens up the possibility to clearly identify the blade and differentiate it from products of other manufacturers.

Since the foil 5 of amorphous metal is applied as cover portion on the strip-shaped carrier member 7 and forms the cutting edge 3 as one cover face of the blade body together with the one-sided ground surface, the position of the cutting edge 3 with respect to the blade body is exactly defined. Thus, this blade structure allows the realization of an extremely exact cutting guidance.

Next, the manufacturing method for the above-described blade is explained.

For the carrier member 7, a carrier tape of about 20 mm width and 200 µm thickness of hardened high-grade steel is used whereas, for the foil 5 of amorphous metal, the above-mentioned soft magnetic material "Vitrovac" of the company Vakuumschmelze of Hanau, Germany is used, said material being available in the form of a metal tape of about 20 mm width and 20 to 25 µm thickness. By a suitable heat treatment, a defined magnetic hysteresis curve has been set previously in the amorphous metal tape in order to provide the metal tape with a magnetic encoding. The carrier tape and the amorphous metal tape are wound up into a coil.

The carrier tape and the amorphous metal tape are wound off the coils to adhere the cover face of the amorphous metal tape having the lower amount of amorphous phase to the carrier tape. To start with, the joining surfaces are prepared for this according to the usual prerequisites, after which a liquid adhesive on cyanoacrylate base is applied. Directly after joining, the two tapes are compressed between two rollers having a defined distance, which leads to an adhesive layer of about 25 to 30 µm thickness and to the strength of the adhesive bond. In the adhesion step, it has to be observed that no heat, which would change the amorphous and soft magnetic properties, is entered into the amorphous metal tape. Moreover, the continuous adhesive layer ensures that the amorphous metal tape is separated from the carrier made of high-grade steel, and the soft magnetic properties of the amorphous metal tape remain substantially unaffected.

For further processing, the multi-layer tape produced in this way, which has the structure shown in Fig. 2, is wound up to form a coil to be subsequently subjected to a punching process. In this punching process, the contour of the blade body and the above-mentioned elongated hole are punched in. The contour of the blade body already corresponds approximately to the final shape of the individual blades of 18.6 mm width and 8 mm length. The punched-out blade bodies remain connected via remaining links, so that the multi-layer tape is not interrupted.

The multi-layer tape having the punched-out blade bodies is then fed to a conventional blade grinding station. The individual blade bodies having the structure shown in Fig. 2 are ground on only one side, i.e. from the side of the carrier material 7. In this way, one obtains the cutting edge area shown in Fig. 1, in which the amorphous metal 5 forms the cutting edge 3.

The grinding process is carried out in three steps with the grinding angle increasing. As shown in Fig. 3, first of all rough grinding with an interior angle of α=5°, then grinding with an interior angle of β=9° and, finally, honing with an interior angle of γ=17° are performed. The final grinding step is performed such that honed section extends only over the amorphous metal 5 to avoid detaching particles from the adhesive layer 9 or the carrier member 7.

Due to the high hardness of the amorphous metal, no burrs as e.g. on conventional steel blades are created, so that it is not necessary to perform counter-honing from the side of the amorphous metal 5. Thus, the blade is noted for a strictly one-sided ground section.

The blade sharpened in this way is further processed like a conventional steel blade, i.e. washed and assembled.

The above-described manufacturing method makes it possible to produce an inexpensive blade of high sharpness.

Besides, in this manufacturing method, the amorphous and soft magnetic properties of the used amorphous metal tape remain essentially unchanged. Since the amorphous metal tape is a product having defined specifications as it is manufactured on an industrial scale, the manufacture-related deviations of the properties of the blade can be controlled considerably more easily than, for example, in conventional blades where the amorphous cutting edge area is achieved by application of an amorphous layer onto a metallic base material or by amorphization of a metallic base material.

Furthermore, the high hardness of the amorphous metal prevents the creation of burrs during the grinding process, so that counter-grinding necessary for removing the burr can be avoided. In this way, the position of the cutting edge 3 with respect to the blade body is exactly defined.

In the following, there is described an example in which the above-described blade is used as microkeratome, i.e. as knife for operations of the cornea.

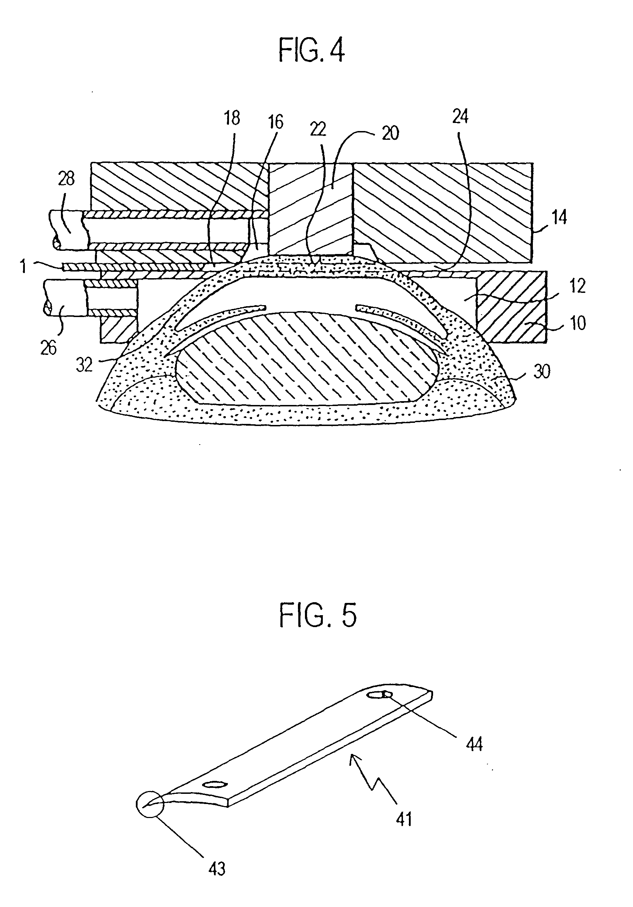

Fig. 4 shows a device for corneal surgery, as it is known from the DE-A-195 40 439. The device shown in the figure serves to sever a lamella from the cornea 32 of an eye 30 by means of a blade 1 in order to expose the stroma, for example for a laser treatment. The device has a positioning ring 10 fixedly mounted on a carrier 14. Said positioning ring 10 rests on the surface of the eye 30 during the operation with an annular chamber 12 being formed between the positioning ring 10 and the eye surface in the area of the eye body, said annular chamber being communicated via a line 26 with a vacuum-generator, said vacuum-generator not being represented in any greater detail. Besides, the carrier 14 forms an annular chamber 16 in the area of the cornea, said annular chamber being likewise communicated via a line 28 with the vacuum-generator. The vacuum in the annular chambers 12, 16 serves to always exactly fix the eye body as well as the cornea lamella to be severed during the cutting process. The blade 1 is guided in a blade guide 18 between the positioning ring 10 and the carrier 14. The movement of the blade 1 in the blade guide 18 is restricted towards the top by an even surface 24 at the lower side of the carrier 14. Besides, on the carrier 14, there is a view glass 20, which may include a reticule to allow an exact alignment of the device with the eye 30. The view glass 20, too, has an even surface 22 at its lower side. The even surface 22 is offset upwards with respect to the even surface 24 of the carrier 14, so that the desired cutting depth for the cornea lamella to be severed, measured from the apex of the cornea perpendicularly into the cornea tissue, can be set. For severing the cornea lamella, the blade 1 is moved with an oscillating movement from left to right.

If, in this example of use, instead of a conventional steel blade the above-described blade according to the invention is used for the blade 1, this has the advantage that the cutting depth into the cornea tissue can be adjusted more exactly. While the cutting edge of a conventional steel blade is formed by counter-grinding, the blade according to the invention is strictly ground on one side only. In this way, the position of the cutting edge with respect to the blade guide 18 and the even surface 24, respectively, is exactly positioned at the lower side of the carrier 14. The cutting depth into the cornea tissue can accordingly be set via the position difference between the even surface 22 of the view glass 20 and the even surface 24 of the carrier 14 without an offset of the blade cutting edge, caused by counter-grinding, having to be considered.

Besides, the device shown in Fig. 4 can be provided with a suitable reading unit by which the magnetic encoding of the blade can be detected and the blade can be clearly identified. This is of particular advantage in practice because surgical blades are often imitated by cut-price suppliers. Some of these so-called "knock-off" products are of such a bad quality that the patient would suffer a damage if they were used. Thus, the described blade offers the advantage that the origin of the blade can be determined exactly. Thus, the installation of a respective reading unit into the device shown in Fig. 4 serves to ensure that only impeccable blades are admitted for being used in an operation.

In addition, also the state of use of the magnetically encoded blade can be detected because the magnetic properties may slightly change due to use, or may be changed intentionally when used. This creates the possibility to prevent a multiple use of the blade.

In the following, a further embodiment of the invention is described.

Fig. 5 shows a perspective view of a blade 41 having a cutting edge 43 made of amorphous metal, which essentially has the same structure as the above-described blade and has been manufactured in a similar way. The blade 41 differs from the blade 1 according to the first embodiment in so far as it is only 3 mm long with a width of about 18.6 mm and is curved towards the side of the foil of amorphous metal in the area of the blade body, i.e. outside the cutting edge area. This serves to increase the rigidity of the blade against deflection. Instead of the elongated hole, the blade body comprises two holes 44 spaced apart from the cutting edge 43 and located at the external surfaces of the blade. The holes 44 serve to receive a support, which is not shown and by means of which the blade 41 can be guided.

To avoid a negative effect on the accuracy of the grinding step, the curvature is only applied to the blade body after sharpening of the blade. When doing this, it has to be taken care that the foil of amorphous metal is not damaged.

The curved blade 41 shown in Fig. 5 is suited as microkeratome. However, when the curved blade 41 is used in the device shown in Fig. 4 for corneal surgery, the device has to be modified such that the blade 41 is guided in a curved blade guide corresponding to the curved shape of the blade instead of in a flat blade guide.

In the following, a third embodiment of the invention is described.

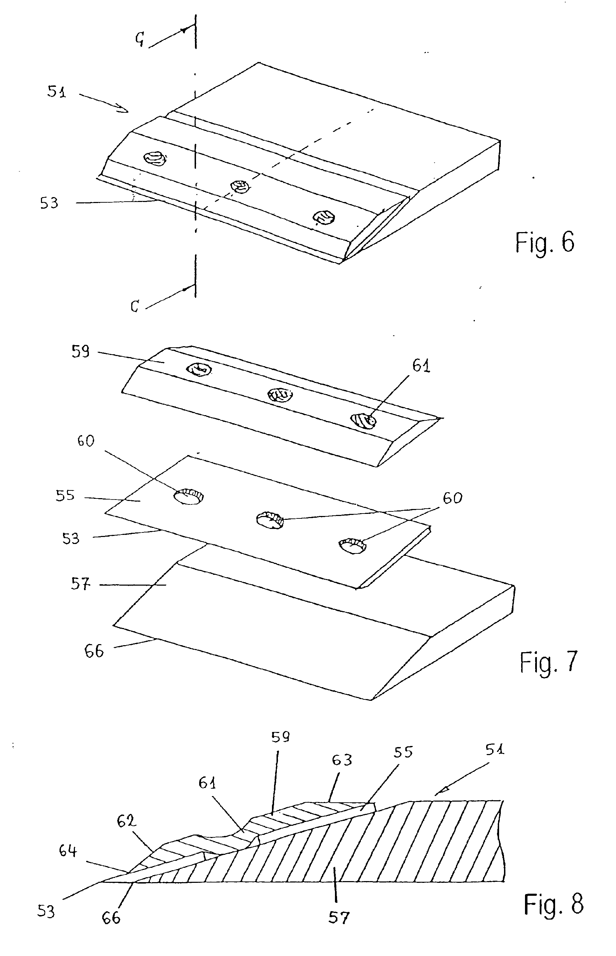

Fig. 6 shows a perspective view of a blade 51 having a cutting edge 53 of amorphous metal. As is shown in detail in the exploded view of Fig. 7, the blade 51 comprises a basically plate-shaped carrier member 57 and a cover member 55 in form of an amorphous metal foil as described in the above embodiments. The cover member 55 is provided with positioning holes 60 disposed in the longitudinal direction of the cutting edge 53. As can furthermore be taken from the sectional view in Fig. 8, a positioning member 59 applied to the cover member 55 has positioning warts or buttons 61 engaging in the positioning holes and being at least partially welded with the carrier member 57. The positioning member 59 which is per se plate-shaped has a supporting edge 64 formed by a front slanting surface 62, said supporting edge being located more closely to the cutting edge 53 than an opposite supporting edge 66 of the carrier member 57. A rear slanting surface 63 of the positioning member 59 is basically formed flush with the upper surface of the blade 51.

An essential advantage of the blade according to the third embodiment is that the use of adhesive for fixing the amorphous cover member is not required, which makes it possible to form the transition from the supporting edge 66 of the carrier member 57 to the cutting edge 53 without the formation of pits due to the grinding out of the softer adhesive material.

The manufacture of the blade 51 according to the third embodiment is effected such that, first of all, a sloping supporting surface of the carrier member 57, shown in Fig. 7, is formed by grinding and subsequent polishing. Then, the cover member 55 existing as the amorphous metal foil and provided with pilot-drilled holes is laid with the cover face having the lower amount of amorphous phase onto this supporting surface and fastened by means of the fixing member 59 by spot welding in the area of the fixing buttons 61 on the carrier member 57. Subsequently, the lower side of the blade shown in Fig. 8 is subjected to surface-grinding, whereby the cutting edge 53 of the amorphous foil is formed. The respective grinding depth serves to set the length of the exposed beveled foil section forming the cutting edge 53 and, in this way, to influence the flexibility of the cutting edge 53 in accordance with the particular field of application of the blade.

The blade 51 shown in Figures 6 to 8 is particularly suited as microkeratome and can advantageously be applied in the device for corneal surgery, shown in Fig. 4.

The invention shall not be restricted to the details of the above-described embodiments.

For the foil of amorphous metal, there can also be used materials which cannot be encoded magnetically. Besides, the thickness of the foil or the amorphous metal tape is not restricted to the 20 to 25 µm mentioned in the first embodiment. In practice, however, only sufficiently amorphous tapes having a thickness ranging from 10 to 50 µm are known according to the present level of knowledge.

The thickness of the carrier strip consisting of hardened high-grade steel is not restricted to the 200 µm used for the first embodiment, but may amount to 150 to 300 µm. Besides, also materials other than hardened high-grade steel may be applied.

The carrier member may have a shape other than a flat strip. Besides the possibility of curving the flat carrier strip, referred to in the case of the second embodiment, the flat carrier strip can also be folded, or curved and/or folded several times. The gain in rigidity which can be achieved thereby can be used to make the blade as such thinner, whereby the penetration behavior of the blade in cutting is improved. In addition, the carrier member can have shape other than the strip shape, such as the wedge shape shown in the third embodiment.

As long as a sufficient adhesive strength is achieved, not only cyanoacrylate but also other adhesives such as, for example, epoxy resin can be used as adhesive in order to join the foil of amorphous metal to the carrier member. However, peeling tests, in which the adhesive capacity of various adhesives were tested, proved cyanoacrylate to have a particularly good adhesive strength. Besides, the thickness of the adhesive layer is not restricted to the 25 to 30 µm mentioned in the first embodiment, but can be set within a range of 5 to 75 µm.

Instead of an adhesive process or a welding process, the foil of amorphous metal may be joined to the carrier member by brazing.

Besides, the blade can also be coated with a nanometer-thin layer of, for example, polymerized silicone. Polymerized silicone is not only particularly biocompatible, but also reduces the friction coefficient of the blade in the cutting process.

Besides the mentioned field of using the blade as microkeratome for corneal operations, the blade is also suited as microtome for preparing tissue samples and as scalpel.

The invention relates to a blade (1) consisting of a carrier member (7) and a foil (5) of amorphous metal joined to the carrier member via an adhesive layer (9), said amorphous metal forming the cutting edge (3) of the blade. The cutting edge (3) of the blade is formed by a one-sided ground section extending over the carrier material as well as the amorphous metal. The use of the foil (5) of amorphous metal allows the manufacture of an inexpensive blade having a high sharpness and a, with respect to the blade body, exactly defined position of the cutting edge (3).

## Claims

1. A blade (1; 41; 51) consisting of a carrier member (7; 57) and a foil (5; 55) of amorphous metal joined to said carrier member, said amorphous metal forming a cutting edge (3; 43; 53).

2. A blade according to claim 1, wherein said foil (5; 55) of amorphous metal is gained from a molten metal by rapid solidification and has a thickness of not more than 50 µm.

3. A blade according to claim 1 or 2, wherein said cutting edge (3; 43; 53) is formed by an one-sided ground section.

4. A blade according to any of claims 1 to 3, said blade having a ground section which extends only over said foil of amorphous metal.

5. A blade according to any of claims 1 to 3, said blade having a multi-faced ground section with the ground surface of the foil (5) of amorphous metal having a greater interior grinding angle (γ) than an adjacent ground surface.

6. A blade according to any of claims 1 to 3, said blade having a multi-faced ground section with the ground surface of the foil of amorphous metal having an offset in respect of an adjacent ground surface.

7. A blade according to any preceding claim, wherein a cover face of said foil (5; 55) of amorphous metal having a higher amount of amorphous phase forms said cutting edge (3; 43; 53).

8. A blade according to claim 1, wherein said foil (5) of amorphous metal is joined to said carrier member (7) via an adhesive layer (9) extending therebetween.

9. A blade according to claim 1, wherein said foil (55) of amorphous metal is mounted on said carrier member (57) by means of a fixing member (59) penetrating said foil (55) and being joined to said carrier member (57).

10. A blade according to claim 1, wherein said carrier member (7; 57) is strip-shaped.

11. A blade according to claim 10, wherein said strip-shaped carrier member (7; 57) is made of hardened high-grade steel.

12. A blade according to claim 11, wherein said strip-shaped carrier member (7; 57) made of hardened high-grade steel has a thickness of 150 to 350 µm.

13. A blade according to claim 10 or 11, wherein said blade is curved or folded alongside said cutting edge (43).

14. A blade according to claim 1, wherein said foil (5) of amorphous metal is soft magnetic and has a remanence ratio of more than 0.7.

15. A method for manufacturing a blade (1; 41), comprising the steps of:
joining a foil (5; 55) of amorphous metal to a carrier member (7; 57) to form a blade body; and
grinding the blade body, so that the amorphous metal forms a cutting edge (3; 43; 53).

16. A method according to claim 15, wherein said cutting edge (3; 43; 53) is formed by grinding the blade body on only one side.

17. A method according to claim 15 or 16, wherein only said foil of amorphous metal is ground.

18. A method according to claim 15, wherein said foil (5) of amorphous metal is joined to said carrier member (7) by adhesion.

19. A method according to claim 15, wherein said foil (5; 55) of amorphous metal and said carrier member (7; 57) are tape-shaped and are joined to form a multi-layer tape from which a plurality of blade bodies (1; 41; 51) is formed by punching in contours.

20. A method according to claim 19, wherein the ground blade body is curved or folded alongside said cutting edge (43).

21. Use of the blade according to any of claims 1 to 14 as a microkeratome, microtome or scalpel.
